# FASCICULE DE BREVET EUROPEEN

(11) **EP 0 325 513 B1**
(45) Date de publication et mention de la délivrance du brevet: **07.04.1993**
(21) Numéro de dépôt: 89400097.5
(22) Date de dépôt: 12.01.1989
(51) Int. Cl.: C07D 233/94, C07D 405/04, A61K 31/415

(54) **Procédé de préparation d'hydroxy-alkyl-1 méthyl-2 nitro-5 imidazoles**
Verfahren zur Herstellung von 1-Hydroxyalkyl-2-methyl-5-nitro-imidazolen
Process for the preparation of 1-hydroxyalkyl-2-methyl-5-nitro-imidazoles

(30) Priorité: 15.01.1988 FR 8800417
(43) Date de publication de la demande: 26.07.1989
(73) Titulaire: RHONE-POULENC SANTE, 92160 Antony (FR)
(72) Inventeur: Buforn, Albert, F-69008 Lyon (FR); Massonneau, Viviane, F-69130 Ecully (FR); Mulhauser, Michel, F-69130 Ecully (FR)
(74) Mandataire: Pilard, Jacques

(56) Documents cités:
- FR-A- 1 212 028
- FR-A- 1 379 787
- FR-A- 1 545 317
- FR-A- 2 073 879
- FR-M- 3 270
- US-A- 3 178 446

## Description

La présente invention concerne un nouveau procédé de préparation d'hydroxyalkyl-1 nitro-5 imidazoles.

Parmi les dérivés de l'imidazole, l'hydroxyéthyl-1 méthyl-2 nitro-5 imidazole (ou métronidazole), l'(hydroxy-2 propyl)-1 méthyl-2 nitro-5 imidazole (ou secnidazole) ou l'(hydroxy-3 propyl)-1 méthyl-2 nitro-5 imidazole (ternidazole) présentent un intérêt tout particulier du fait de leurs propriétés thérapeutiques remarquables.

Il est connu de préparer le métronidazole par action d'un excès d'oxyde d'éthylène sur le méthyl-2 nitro-4 (ou -5) imidazole dans les conditions décrites dans le brevet français FR 1 379 915. Cependant les rendements ne sont pas satisfaisants.

Il est connu de préparer le (fluoro-4 phényl)-2 hydroxyméthyl-1 nitro-5 imidazole par action du sulfate d'éthylène sur le (fluoro-4 phényl)-2 nitro-4 (ou -5) imidazole selon le procédé décrit dans le brevet américain US 3 743 653. Cependant dans ce cas le rendement est inférieur à 10 %.

Dans le brevet américain US 3 178 446 est décrite la condensation du carbonate d'éthylène sur le méthyl-2 nitro-5 imidazole conduisant essentiellement à l'hydroxyéthyl-1 nitro-4 méthyl-2 imidazole.

Il a maintenant été trouvé, et c'est ce qui fait l'objet de la présente invention, que les hydroxyalkyl-1 nitroimidazoles de formule générale : 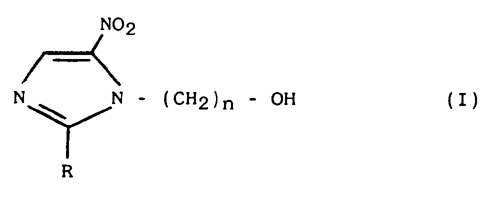
dans laquelle R représente un atome d'hydrogène ou un radical alkyle contenant 1 à 4 atomes de carbone ou alkényle contenant 2 à 4 atomes de carbone, les radicaux alkyle et alkényle étant éventuellement substitués par un ou plusieurs radicaux, identiques ou différents, choisis parmi les radicaux phényles, phénoxy ou hétérocycliques oxygénés à 5 ou 6 chaînons, ou bien un radical aryle contenant 6 à 10 atomes de carbone éventuellement substitué par un ou plusieurs atomes ou radicaux, identiques ou différents, choisis parmi les atomes d'halogène et les radicaux alkyles contenant 1 à 4 atomes de carbones, alkoxy dont la partie alkyle contient 1 à 4 atomes de carbone, phényle, phénoxy ou nitro, ou bien un radical cycloalkyle contenant 5 ou 6 atomes de carbone, les radicaux phényles, phénoxy ou hétérocycliques pouvant être éventuellement substitués par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro et n est égal à 2 ou 3 et un des atomes de la chaîne alkylène peut être substitué par un radical méthyle, peuvent être obtenus avec de bons rendements par action d'un acide fort en présence du diacétate d'un alkylènediol de formule générale :

HO - (CH₂)ₙ - OH (II)

dans laquelle n est égal à 2 ou 3 et un des atomes de carbone de la chaîne alkylène peut être substitué par un radical méthyle, sur un dérivé de l'imidazole de formule générale : 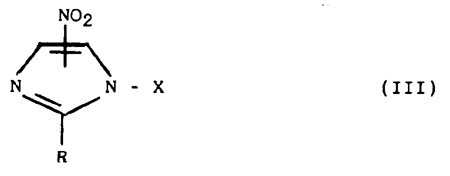
dans laquelle R est définie comme précédemment et X représente un radical éliminable par hydrolyse ou alcoolyse tel qu'un radical hydroxyméthyle, alcoxyméthyle dont la partie alcoyle contient 1 à 4 atomes de carbone ou acyloxyméthyle dont la partie acyle contient 1 à 4 atomes de carbone en chaîne droite ou ramifiée ou un radical éthylénique allylique, tel que le radical allyle, ou un radical arylméthyle tel que le radical benzyle, suivie de l'hydrolyse ou de l'alcoolyse du produit de condensation ainsi obtenue.

Plus particulièrement, la présente invention concerne la préparation de l'hydroxyéthyl-1 méthyl-2 nitro-5 imidazole et de l'(hydroxy-2 propyl)-1 méthyl-2 nitro-5 imidazole.

Généralement, l'acide fort est choisi parmi les acides sulfurique, méthanesulfonique ou p.toluènesulfonique.

Généralement, la condensation est effectuée à une température comprise entre 80 et 140°C en utilisant une mole d'acide et une mole d'ester par mole de dérivé de l'imidazole de formule (III) mis en oeuvre.

Par ailleurs, lorsqu'on l'on utilise le diacétate de l'alkylèneglycol de formule générale (II), il est particulièrement avantageux d'utiliser un acide deshydraté par exemple au moyen d'anhydride acétique et en distillant l'acide acétique formé.

Le produit de condensation peut être solubilisé :
- soit dans une solution aqueuse d'un acide minéral fort, par exemple l'acide sulfurique ou l'acide chlorhydrique,
- soit dans un alcool tel que, par exemple, le méthanol ou l'éthanol.

Lorsque le produit de condensation est solubilisé dans l'eau acidifiée, l'hydroxyalkyl-1 nitroimidazole est extrait selon les techniques habituelles après alcalinisation du mélange réactionnel à un pH voisin de 10.

Lorsque le produit de condensation est solubilisé dans un alcool, l'hydroxyalkyl-1 nitroimidazole est isolé selon les techniques habituelles sans traitement préalable du mélange réactionnel.

Pour la mise en oeuvre du procédé, il n'est pas nécessaire d'isoler le produit de condensation intermédiaire, l'hydrolyse ou l'alcoolyse pouvant être enchaînées dans le même appareil.

Le dérivé de l'imidazole de formule générale (III) dans laquelle X représente un radical hydroxyméthyl, méthoxyméthyl ou alcoxyméthyle ou acyloxyméthyle peut être préparé dans les conditions décrites dans le brevet anglais GB 1 026 631.

Les exemples suivants, donnés à titre non limitatif, montrent comment l'invention peut être mise en pratique.

### EXEMPLE 1

Dans un appareil à distiller, dont le récepteur est plongé dans un bain acétone - carboglace, on introduit 4,38 g de diacétate d'éthylène glycol (0,03 mole), 2 g d'accétoxyméthyl-1 méthyl-2 nitro-4 imidazole (0,02 mole), 1,56 g d'acide sulfurique concentré (d = 1,83) (0,016 mole) et 0,45 cm3 d'anhydrique acétique afin d'éliminer l'eau présente dans l'acide sulfurique. On établit dans l'appareil, une pression de 150 mm de mercure (20 kPa), puis on chauffe le mélange réactionnel pendant 8 heures à 95°C. Pendant le chauffage, on distille 0,94 g d'acide acétique. L'appareil à distiller est remis sous pression atmosphérique et après refroidissement de la masse réactionnelle, on ajoute 15 cm3 d'éthanol dans le bouilleur. L'appareil à distiller est remplacé par un réfrigérant. La solution obtenue après addition de l'éthanol est chauffée au reflux pendant 4 heures.

Après refroidissement, on dose par CLHP avec étalonnage externe, 1 g de métronidazole dans le mélange réactionnel.

Le taux de transformation de l'acétoxyméthyl-1 méthyl-2 nitro-4 imidazole est de 71%.

Le rendement en métronidazole est de 59% par rapport à l'acétoxyméthyl-1 méthyl-2 nitro-4 imidazole mis en oeuvre et de 83% par rapport à l'acétoxyméthyl-1 méthyl-2 nitro-4 imidazole transformé.

### EXEMPLE 2

Dans un ballon muni d'une agitation, on introduit 12 g (0,06 mole) d'acétoxyméthyl-1 nitro-4 imidazole, 26 g (0,18 mole) de diacétate de glycol et 9,4 g (0,09 mole) d'acide sulfurique concentré. Le mélange est chauffé pendant 6 heures sous une pression de 150 mm de mercure (20 kPa). Pendant le chauffage, on distille un mélange d'acide acétique et de diacétate de glycol. On ajoute 30 cm3 d'eau puis chauffe au reflux pendant 4 heures.

Après dilution, le dosage par chromatographie liquide à haute performance (CLHP) avec étalonnage externe montre que :
- le taux de transformation de l'acétoxyméthyl-1 nitro-4 imidazole est de 87,8%
- le rendement en hydroxyéthyl-1 nitro-5 imidazole est de 85% par rapport à l'acétoxyméthyl-1 nitro-4 imidazole transformé.

## Revendications

1. Procédé de préparation d'un hydroxyalkyl-1 nitroimidazole de formule générale : 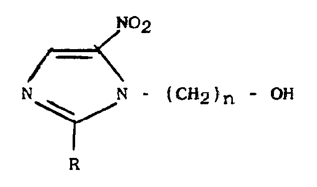 dans laquelle R représente un atome d'hydrogène ou un radical alkyle contenant 1 à 4 atomes de carbone ou alkényle contenant 2 à 4 atomes de carbone, les radicaux alkyle et alkényle étant éventuellement substitués par un ou plusieurs radicaux, identiques ou différents, choisis parmi les radicaux phényles, phénoxy ou hétérocycliques oxygénés à 5 ou 6 chaînons, ou bien un radical aryle contenant 6 à 10 atomes de carbone éventuellement substitué par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro, ou bien un radical cycloalkyle contenant 5 ou 6 atomes de carbone, les radicaux phényle, phénoxy ou hétérocycliques pouvant être éventuellement substitués par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro et n est égal à 2 ou 3 et un des atomes de carbone de la chaîne alkylène peut être substitué par un radical méthyle, caractérisé en ce que l'on fait agir un acide fort en présence du diacétate d'un alkylènediol de formule générale :
HO - (CH₂)ₙ - OH
dans laquelle n est égal à 2 ou 3 et un des atomes de carbone de la chaîne alkylène peut être substitué par un radical méthyle, sur un dérivé de l'imidazole de formule générale : 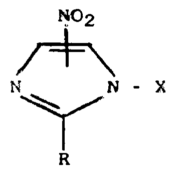 dans laquelle R est défini comme précédemment et X représente un radical éliminable par hydrolyse ou alcoolyse tel que le radical hydroxyméthyle, alcoxyméthyle dont la partie alcoyle contient 1 à 4 atomes de carbone ou acyloxyméthyle dont la partie acyle contient 1 à 4 atomes de carbone en chaîne droite ou ramifiée ou un radical éthylénique allylique ou un radical arylméthyle, à une température comprise entre 80 et 140°C, puis hydrolyse ou alcoolyse le produit de condensation obtenu et isole l'hydroxyalkyl-1 nitroimidazole.

2. Procédé selon la revendication 1 caractérisé en ce que l'acide fort est choisi parmi les acides sulfurique, méthanesulfonique et p.toluènesulfonique.

3. Procédé selon la revendication 1 caractérisé en ce que l'on utilise au moins une mole d'acide fort et au moins une mole de diester par mole de dérivé de l'imidazole mis en oeuvre.

4. Procédé selon la revendication 1 caractérisé en ce que l'hydrolyse du produit de condensation est effectuée en présence d'un acide fort choisi parmi l'acide sulfurique et l'acide chlorhydrique.

5. Procédé selon la revendication 1 caractérisé en ce que l'alcoolyse est effectuée par chauffage en présence d'un alcool choisi parmi le méthanol et l'éthanol.

6. Procédé selon la revendication 1 caractérisé en ce que l'hydroxyalkyl-1 nitroimidazole obtenu est le métronidazole, le secnidazole ou le ternidazole.

## Patentansprüche

1. Verfahren zur Herstellung eines 1-Hydroxyalkyl-nitroimidazols der allgemeinen Formel 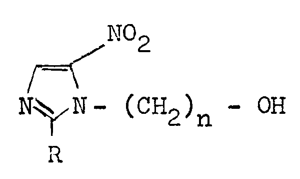 worin R für ein Wasserstoffatom oder einen Alkylrest mit 1 bis 4 Kohlenstoffatomen oder einen Alkenylrest mit 2 bis 4 Kohlenstoffatome, wobei die Alkyl- und Alkenylreste gegebenenfalls durch einen oder mehrere gleiche oder verschiedene Reste substituiert sind, ausgewählt unter den Phenyl-, Phenoxy- oder sauerstoffhaltigen, heterocyclischen Resten mit 5 oder 6 Kettengliedern, oder für einen Arylrest mit 6 bis 10 Kohlenstoffatomen, der gegebenenfalls durch ein oder mehrere gleiche oder verschiedene Atome oder Reste substituiert ist, ausgewählt unter den Halogenatomen und den Alkylresten mit 1 bis 4 Kohlenstoffatomen, den Alkoxyresten, deren Alkylteil 1 bis 4 Kohlenstoffatome aufweist, den Phenyl-, Phenoxy- oder Nitrogruppen, oder für einen Cycloalkylrest mit 5 oder 6 Kohlenstoffatomen, wobei die Phenyl-, Phenoxy- oder heterocyclischen Reste gegebenenfalls substituiert sein können durch ein oder mehrere gleiche oder verschiedene Atome oder Reste, ausgewählt unter den Halogenatomen und den Alkylresten mit 1 bis 4 Kohlenstoffatomen, den Alkoxyresten, deren Alkylteil 1 bis 4 Kohlenstoffatome aufweist, den Phenyl-, Phenoxy- oder Nitrogruppen, steht und n 2 oder 3 bedeutet und eines der Kohlenstoffatome der Alkylenkette durch einen Methylrest substituiert sein kann, dadurch **gekennzeichnet,** daß man eine starke Säure in Gegenwart des Diacetats eines Alkylendiols der allgemeinen Formel
HO - (CH₂)ₙ - OH
worin n 2 oder 3 bedeutet und eines der Kohlenstoffatome der Alkylenkette durch einen Methylrest substituiert sein kann, mit einem Imidazolderivat der allgemeinen Formel 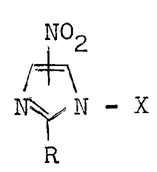 worin R wie vorstehend definiert ist und X einen durch Hydrolyse oder Alkoholyse entfernbaren Rest, wie den Hydroxymethylrest, den Alkoxymethylrest, dessen Alkylteil 1 bis 4 Kohlenstoffatome aufweist, oder den Acycloxymethylrest, dessen Acylteil 1 bis 4 Kohlenstoffatome in gerader oder verzweigter Kette aufweist,oder einen allylischen Ethylenrest oder einen Arylmethylrest bedeutet, bei einer Temperatur zwischen 80 und 140°C umsetzt, danach das erhaltene Kondensationsprodukt hydrolysiert oder alkoholisiert und das 1-Hydroxyalkyl-nitroimidazol isoliert.

2. Verfahren gemäß Anspruch 1, dadurch gekennzeichnet, daß die starke Säure unter Schwefelsäure, Methansulfonsäure und p-Toluolsulfonsäure ausgewählt wird.

3. Verfahren gemäß Anspruch 1, dadurch gekennzeichnet, daß man zumindest ein Mol der starken Säure und zumindest ein Mol des Diester je Mol des eingesetzten Imidazolderivats verwendet.

4. Verfahren gemäß Anspruch 1, dadurch gekennzeichnet, daß die Hydrolyse des Kondensationsprodukts in Gegenwart einer starken Säure, ausgewählt unter Schwefelsäure und Chlorwasserstoffsäure, durchgeführt wird.

5. Verfahren gemäß Anspruch 1, dadurch gekennzeichnet, daß die Alkoholyse durch Erhitzen in Gegenwart eines Alkohols, ausgewählt unter Methanol und Ethanol, durchgeführt wird.

6. Verfahren gemäß Anspruch 1, dadurch gekennzeichnet, daß das erhaltene 1-Hydroxyalkyl-nitroimidazol das Metronidazol, Secnidazol oder Ternidazol ist.

## Claims

1. Process for the preparation of 1-(hydroxyalkyl)-nitroimidazole of general formula: 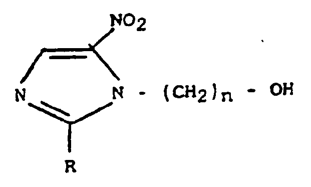 in which R represents a hydrogen atom or an alkyl, containing 1 to 4 carbon atoms, or alkenyl, containing 2 to 4 carbon atoms, radical, the alkyl and alkenyl radicals being optionally substituted with one or more radicals, which are identical or different, chosen-from the phenyl, phenoxy or oxygen-containing, 5- or 6-membered heterocyclic radicals , or else an aryl radical containing 6 to 10 carbon atoms optionally substituted with one or more atoms or radicals, which are identical or different, chosen from the halogen atoms and the alkyl, containing 1 to 4 carbon atoms, alkoxy, the alkyl part of which contains 1 to 4 carbon atoms, phenyl, phenoxy or nitro radicals, or else a cycloalkyl radical containing 5 or 6 carbon atoms, it being possible for the phenyl, phenoxy or heterocyclic radicals to be optionally substituted with one or more atoms or radicals, which are identical or different, chosen from the halogen atoms and the alkyl, containing 1 to 4 carbon atoms, alkoxy, the alkyl part of which contains 1 to 4 carbon atoms, phenyl, phenoxy or nitro radicals and n is equal to 2 or 3 and one of the carbon atoms of the alkylene chain can be substituted with a methyl radical, characterised in that a strong acid is reacted in the presence of the diacetate of an alkylenediol of general formula:
HO- (CH₂)ₙ-OH
in which n is equal to 2 or 3 and one of the carbon atoms of the alkylene chain can be substituted with a methyl radical, with an imidazole derivative of general formula: 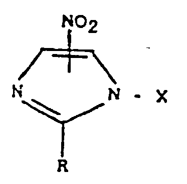 in which R is defined as above and X represents a radical which can be removed by hydrolysis or alcoholysis, such as the hydroxymethyl, alkoxymethyl, the alkyl part of which contains 1 to 4 carbon atoms, or acyloxymethyl, the acyl part of which contains 1 to 4 straight- or branched-chain carbon atoms, radical or an allyl-like ethylenic radical or an arylmethyl radical, at a temperature of between 80 and 140°C, the condensation product obtained is then hydrolysed or alcoholysed and the 1-(hydroxyalkyl)nitroimidazole is isolated.

2. Process according to Claim 1, characterised in that the strong acid is chosen from sulphuric, methanesulphonic and p-toluenesulphonic acids.

3. Process according to Claim 1, characterised in that at least one mole of strong acid and at least one mole of diester are used per mole of imidazole derivative used.

4. Process according to Claim 1, characterised in that the hydrolysis of the condensation product is carried out in the presence of a strong acid chosen from sulphuric acid and hydrochloric acid.

5. Process according to Claim 1, characterised in that the alcoholysis is carried out by heating in the presence of an alcohol chosen from methanol and ethanol.

6. Process according to Claim 1, characterised in that the 1-(hydroxyalkyl)nitroimidazole obtained is metronidazole, secnidazole or ternidazole.
